# EUROPÄISCHE PATENTSCHRIFT

(11) **EP 0 709 666 B1**
(45) Veröffentlichungstag und Bekanntmachung des Hinweises auf die Patenterteilung: **29.10.1997**
(21) Anmeldenummer: 95114552.3
(22) Anmeldetag: 15.09.1995
(51) Int. Cl.: G01N 1/20, A01J 5/04

(54) **Vorrichtung zum Entnehmen einer repräsentativen Milchprobe**
Device for withdrawing a representative milk sample
Dispositif pour la prise d'un échantillon représentatif de lait

(30) Priorität: 28.10.1994 DE 4438434
(43) Veröffentlichungstag der Anmeldung: 01.05.1996
(73) Patentinhaber: JANSKY GMBH, D-48282 Emsdetten (DE)
(72) Erfinder: Jansky, Manfred, Dipl.-Ing., D-48282 Emsdetten (DE)
(74) Vertreter: Cohausz & Florack

(56) Entgegenhaltungen:
- EP-A- 0 420 800
- DE-A- 1 798 275
- DE-A- 3 222 234
- DE-A- 3 502 858
- DE-A- 3 931 956

## Beschreibung

Die Erfindung betrifft eine Vorrichtung zur Entnahme einer Probe in einer Milchsammelanlage mit einer von einer Förderleitung abzweigenden und zu einem Probengefäß führenden Entnahmeleitung sowie einer einstellbaren Dosierpumpe.

Um von der von einem Milchproduzenten angenommenen Milch eine repräsentative Probe zu erhalten, ist es erforderlich, während der gesamten Annahme aus der Förderleitung mengenproportional Milch abzuzweigen. Da das von den Produzenten zur Verfügung gestellte Milchvolumen in der Größenordnung bis zu zwei Zehnerpotenzen schwankt, die zu entnehmende Probe aber immer das gleiche Volumen, beispielsweise 30 bis 40 ccm, haben soll, ist es üblich, im Fördervolumen einstellbare Dosierpumpen einzusetzen. Die Einstellung des jeweiligen Fördervolumens bei dem einzelnen Produzenten erfolgt entweder nach Abschätzung des zur Verfügung gestellten Volumens von Hand oder automatisch aufgrund der abgespeicherten Volumina dieses Produzenten aus den letzten Liefertagen.

Die Repräsentativität der Milchprobe kann beeinträchtigt werden, wenn sich in der abzweigenden Entnahmeleitung und in der Dosierpumpe noch Milchreste von der letzten Probenahme finden. Um Verfälschungen durch solche Milchreste, sogenannte Verschleppungsfehler, möglichst klein zu halten, ist es bekannt, ganz am Anfang der Förderung von Milch über die Förderleitung mittels der Dosierpumpe eine Rückspülung vorzunehmen. Bei großen Milchvolumina mag der mit einer solchen Rückspülung verbundene Nachteil, daß ein Teil der Milch zu Beginn der Annahme für die Bildung der Probe der Milch verlorengeht, vernachlässigbar sein, das gilt jedoch nicht für kleine anzunehmende Milchvolumina.

Aus der DE 35 02 858 A1 ist eine Vorrichtung zur Entnahme einer Milchprobe bekannt, bei der das geschätzte Volumen vor Beginn der Milchannahme in einen Steuerrechner eingegeben wird, der aus dieser Vorgabe die Förderleistung einer Peristaltikpumpe berechnet und einstellt, sowie darüber hinaus ein Stellglied steuert, das die Entnahmeleitung taktweise über das Probengefäß beziehungsweise über einen Ablauftrichter schwenkt.

Bei diesem Stand der Technik wird die Leitungslänge der Milchprobenentnahmevorrichtung auf ein Mindestmaß reduziert, um somit die Menge von an der Leitungswand haftenden Milchresten zu verringern. Trotzdem können aufgrund des geringen Probenvolumens Verschleppungsfehler auftreten, die bei geringen anzunehmenden Milchvolumina die Repräsentativität der Milchprobe erheblich beeinträchtigen.

Aus der EP-98966 A2 ist eine Milchprobenentnahmevorrichtung bekannt, bei der während des gesamten Annahmevorgangs ein Teilstrom aus dem Hauptstrom abgezweigt und in einen kleinen Sammel- und Mischbehälter gefüllt wird, aus dem am Ende des Annahmevorganges ein noch kleineres Volumen als Milchprobe entnommen wird. Nach dem Erstellen der Probe wird die übrige im Sammel- und Mischbehälter verbleibende Milch abgelassen und der bereits angenommenen Milch zugegeben. Beim nächsten Produzenten wird in gleicher Weise verfahren, also der Sammel- und Mischbehälter während des Annahmevorgangs mit Milch gefüllt. Um die Verfälschung durch Milchprobenreste möglichst klein zu halten, läßt sich das Abzweigventil der Milchprobenentnahmevorrichtung so einstellen, daß der Sammel- und Mischbehälter während einer Annahme annähernd vollständig gefüllt wird, so daß das Verhältnis von für die Probe entnommener Milch und Restmilch möglichst groß und damit der Verschleppungsfehler möglichst gering wird. Verbleibende Milchreste können durch einen Spülvorgang aus dem Leitungssystem entfernt werden.

Nachteilig ist auch bei diesem Stand der Technik, daß durch im Leitungssytsem verbleibende Milchreste Verschleppungsfehler auftreten können, die zwar durch den Sammel- und Mischvorgang vermindert werden, aber erst durch einen mit erhöhtem technischen und zeitlichen Aufwand verbundenen Spülvorgang vollständig vermieden werden können.

Letztlich ist aus der CH-PS 677 537 ein Verfahren zum Entnehmen einer Milchprobe bekannt, bei dem eine von einer Förderleitung zu einer Entnahmeflasche führende Entnahmeleitung vor der Probenentnahme mit der Milch des aktuellen Lieferanten durchgespült wird, um Milchprobenreste von vorherigen Lieferanten aus dem Entnahmesystem zu beseitigen. Bei diesem Entnahmesystem wird die Milch über eine Entnahmeleitung von einer Entnahmepumpe direkt in das Milchprobengefäß geleitet. Die Repräsentativität der Milchprobe soll dadurch gewährleistet werden, daß die Förderleistung der Pumpe der voraussichtlichen Milchabnahmemenge angepaßt wird, so daß während der gesamten Milchabnahme ein Milchprobestrom entnommen wird. Der Durchspülvorgang vor der Probenentnahme soll so vollzogen werden, daß die Entnahmeleitung und die damit verbundene, in das Probengefäß hineinreichende Injektionsnadel mit Milch gefüllt werden und nach dem vollständigen Füllen durch Rückpumpen der Milch in die Förderleitung wieder entleert werden, ohne daß dabei Milch in das Probengefäß gelangt.

Vor dem Hintergrund dieses Standes der Technik liegt der Erfindung die Aufgabe zugrunde, eine Vorrichtung der eingangs genannten Art zu schaffen, bei der die Repräsentativität der Milchproben bei gleichzeitig geringem Geräteaufwand verbessert wird.

Diese Aufgabe wird dadurch gelöst, daß von der Entnahmeleitung eine Leitung, in der die Dosierpumpe liegt, zu einem Mischbehälter abzweigt, daß die Förderrichtung der Dosierpumpe umkehrbar ist und daß der für die Milchprobe zu entnehmende Milchstrom von einer in der Entnahmeleitung angeordneten Armaturenanordnung in einer ersten Phase aus der Förderleitung in den Mischbehälter und in einer zweiten Phase von dem Mischbehälter in das Probengefäß lenkbar ist.

Durch die vorliegende Erfindung wird der Verschleppungsfehler zum einen durch die Verringerung der im Leitungssystem befindlichen Milchrestmenge, zum anderen durch die Verminderung ihres Einflusses auf die Repräsentativität der Milchprobe erheblich verringert. Da nämlich die Dosierpumpe in zwei Förderrichtungen betrieben wird, wird ein Teil des Leitungssystems in doppelter Funktion zum einen als Zulauf für den Mischbehälter, zum anderen als Leitung von dem Mischbehälter zur Probenflasche genutzt. Somit ist die Länge des Leitungssystems der Milchprobenentnahmevorrichtung und dementsprechend die benetzbare Oberfläche in diesem System auf ein Minimum reduziert, so daß die Menge der in der Milchprobenentnahmevorrichtung verbleibenden Milchreste erheblich verringert wird. Der Einfluß der Milchrestmenge auf Verschleppungsfehler wird dadurch reduziert, daß durch das Sammeln und Mischen der entnommenen Milch für die Milchprobe eine große Menge Milch für die Meßprobe zur Verfügung steht, so daß im Vergleich zu der Konzentration von Restmilch bei der direkten Abfüllung der entnommenen Milch in ein Probengefäß bei der vorliegenden Vorrichtung die Konzentration von Restmilch im Mischbehälter - und damit auch in der Milchprobe - sehr gering ist.

Obwohl ein großer Aufwand für die Reduzierung von Verschleppungsfehlern betrieben wird, ist der konstruktive Aufbau der erfindungsgemäßen Vorrichtung denkbar einfach. Durch den Einsatz einer Dosierpumpe mit umkehrbarer Förderrichtung kann die Leitung für die Zufuhr von Milch in den Mischbehälter gleichzeitig die Funktionen der Abfuhr der Milchprobe als auch der Rückführung der restlichen Milch aus dem Mischbehälter übernehmen.

Die erfindungsgemäße Vorrichtung läßt verschiedene Möglichkeiten der Ausführung zu. So läßt sich beispielsweise die Armaturenanordnung durch zwei einfache Ventile, durch ein Mehrwegventil oder ähnliche gleichwirkende Mittel gestalten.

Ebenfalls vorteilhaft ist es, wenn eine Rückführleitung von dem Mischbehälter zu der als Saugförderleitung ausgebildeten Förderleitung führt, so daß auf einfache Weise der Mischbehälter und das Leitungssystem der Milchprobenentnahmevorrichtung von Milchresten entleert werden können und diese Milchreste der angenommenen Milch zugeführt werden. Diese Rückführleitung kann über ein Ventil mit dem Mischbehälter oder aber über ein Mehrwegventil außerdem mit der Leitung, in der die Dosierpumpe liegt, verbunden sein.

Als besonders geeignete Dosierpumpen haben sich Peristaltikpumpen erwiesen, da sie eine sehr konstante Förderrate bei sehr unterschiedlichen Fördervolumina ermöglichen.

Im folgenden wird die Erfindung anhand einer Zeichnung näher erläutert, die schematisch eine Ausführungsform der Probenentnahmevorrichtung mit einem Teil einer Milchannahmeanlage zeigt.

In einer Milchsammelanlage mit einem Sammeltank, einer Volumenmeßeinrichtung und einem Luftabscheider ist lediglich der Probenabzweig 1 an einer als Saugförderleitung ausgebildeten Förderleitung 2 gezeigt. An dem Probenabzweig 1 zweigt eine Entnahmeleitung 3 zu dem Probengefäß 4 ab. In der Entnahmeleitung 3 sind zwei Ventile 5a,5b vorgesehen, zwischen denen eine Leitung 6 abzweigt, die über ein Mehrwegventil 7 mit einem Mischbehälter 8 mit einem Mischelement 8a verbunden ist. In der Leitung 6 liegt eine in der Förderrichtung umschaltbare Peristaltikpumpe 9. Der Mischbehälter 8 ist über das Mehrwegventil 7 und eine Rückführleitung 10 mit der Förderleitung 2 verbunden.

Die beschriebene Probenentnahmevorrichtung arbeitet auf folgende Art und Weise:

Zu Beginn der Probenentnahme ist das Ventil 5a geöffnet, das Ventil 5b geschlossen und das Mehrwegventil 7 befindet sich in einer ersten Stellung, so daß Milch aus der Saugförderleitung 2 durch die Peristaltikpumpe 9 über einen Teil der Entnahmeleitung 3 und über die Leitung 6 in den Mischbehälter 8 gefördert wird. Ist die Annahme der Milch beendet, läuft die Peristaltikpumpe 9 noch solange weiter, bis die Leitungen 3,6 leergesaugt sind. Nachdem die Milch im Mischbehälter 8 gemischt worden ist, werden das Ventil 5a geschlossen und das Ventil 5b geöffnet und die Förderrichtung der Peristaltikpumpe 9 umgekehrt. So kann die entnommene Milch aus dem Mischbehälter 8 über die Leitung 6 und den anderen Teil der Entnahmeleitung 3 in das Probengefäß 4 gefördert werden. Wenn das Probengefäß 4 gefüllt ist, wird die Förderrichtung der Peristaltikpumpe 9 erneut umgekehrt, damit bei einer zweiten Stellung des Mehrwegventils 7 die Restmilch in den Leitungen 3,6 in die Rückführleitung 10 gepumpt und von dem dort anliegenden Sog in die Saugförderleitung abgeführt wird. Gleichzeitig wird bei dieser zweiten Stellung des Mehrwegventils 7 die Restmilch in dem Mischbehälter 8 von dem Sog durch die Rückführleitung in die Förderleitung 2 zurückgeführt.

In einer nicht dargestellten alternativen Ausführung kann auf das Mehrwegventil 7 und die Rückführleitung 10 verzichtet werden. In dieser Ausführung wird, nachdem das Probengefäß 4 gefüllt ist, das Ventil 5b geschlossen und das Ventil 5a geöffnet, so daß die Restmilch in dem Mischbehälter 8 und die in den Leitungen 3,6 verbliebene Restmilch durch die Peristaltikpumpe 9 in die Förderleitung zurückgepumpt wird.

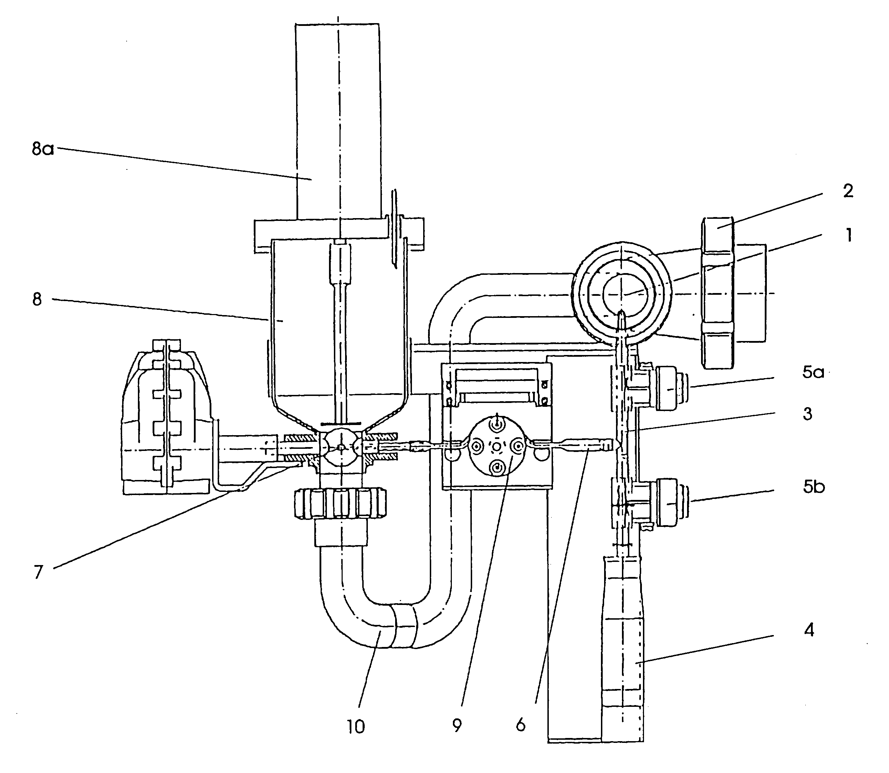

## Patentansprüche

1. Vorrichtung zur Entnahme einer Probe in einer Milchsammelanlage, bestehend aus einer von einer Förderleitung (2) abzweigenden und zu einem Probengefäß (4) führenden Entnahmeleitung (3) sowie einer einstellbaren Dosierpumpe (9), wobei die Förderrichtung der Dosierpumpe (9) umkehrbar ist,
**dadurch gekennzeichnet**,
daß von der Entnahmeleitung (3) eine Leitung (6), in der die Dosierpumpe (9) liegt, zu einem Mischbehälter (8) abzweigt, und daß der für die Milchprobe zu entnehmende Milchstrom von einer in der Entnahmeleitung (3) angeordneten Armaturenanordnung (5a,5b) in einer ersten Phase aus der Förderleitung (2) in den Mischbehälter (8) und in einer zweiten Phase von dem Mischbehälter (8) in das Probengefäß (4) lenkbar ist.

2. Vorrichtung nach Anspruch 1,
**dadurch gekennzeichnet**, daß in der Entnahmeleitung (3) vor und nach der Abzweigung der Leitung (6) je ein Ventil (5a,5b) angeordnet ist.

3. Vorrichtung nach Anspruch 1,
**dadurch gekennzeichnet**, daß die Armaturenanordnung (5a,5b) ein Mehrwegventil ist und die Leitung (6) über das Mehrwegventil (7) mit der Entnahmeleitung (3) verbunden ist.

4. Vorrichtung nach einem der Ansprüche 1 bis 3,
**dadurch gekennzeichnet,** daß die Förderleitung (2) eine Saugförderleitung ist.

5. Vorrichtung nach Anspruch 4,
**dadurch gekennzeichnet,** daß eine Rückführleitung (10) von dem Mischbehälter (8) über ein Ventil (7) mit der Saugförderleitung (2) verbunden ist.

6. Vorrichtung nach Anspruch 5,
**dadurch gekennzeichnet,** daß das Ventil (7) ein Mehrwegventil ist und die Leitung (6), der Mischbehälter (8) und die Rückführleitung (10) über das Ventil (7) miteinander verbunden sind.

7. Vorrichtung nach einem der Ansprüche 1 bis 6,
**dadurch gekennzeichnet,** daß die Dosierpumpe (9) eine Peristaltikpumpe ist.

## Claims

1. An apparatus for the taking of a sample in a milk collecting installation, comprising a sampling line (3) branching off from a delivery line (2) and extending to a sample vessel (4) and an adjustable metering pump (9), wherein the delivery direction of the metering pump (9) can be reversed,
characterized in that a line (6) in which the metering pump (9) is disposed branches off from the sampling line (3) to a mixing tank (8), and the flow of milk to be taken for the sample can be guided in a first phase by a valve arrangement (5a, 5b) disposed in the sampling line (3) from the delivery line (2) to the mixing tank, and in a second phase from the mixing container (8) to the sample vessel (4).

2. An apparatus according to claim 1,
characterized in that a valve (5a, 5b) is disposed in the sampling line (3) respectively upstream and downstream of the branching-off of the line (6).

3. An apparatus according to claim 1,
characterized in that the valve arrangement (5a, 5b) is a multiway valve and the line (6) is connected via the multiway valve (7) to the sampling line (3).

4. An apparatus according to one of claims 1 to 3,
characterized in that the delivery line (2) is a suction delivery line.

5. An apparatus according to claim 4,
characterized in that a return line (10) from the mixing tank (8) is connected via a valve (7) to the suction delivery line (2).

6. An apparatus according to claim 5,
characterized in that the valve (7) is a multiway valve and the line (6), the mixing tank (8) and the return line (10) are connected to one another via the valve (7).

7. An apparatus according to one of claims 1 to 6,
characterized in that the metering pump (9) is a peristaltic pump.

## Revendications

1. Dispositif de prélèvement d'un échantillon dans une installation de collecte de lait, constitué d'une conduite de prélèvement (3) piquée sur une conduite de transport (2) et conduisant à un récipient d'échantillonnage (4), ainsi que d'une pompe de dosage réglable (9), le sens de circulation de la pompe de dosage (9) étant inversible, caractérisé en ce qu'une conduite (6), dans laquelle se trouve la pompe de dosage (9), est piquée sur la conduite de prélèvement (3) et conduit à un récipient de mélange (8), et en ce que le courant de lait à prélever pour constituer l'échantillon de lait peut être dirigé, par une robinetterie (5a, 5b) disposée dans la conduite de prélèvement (3), dans une première phase de la conduite de transport (2) jusque dans le récipient de mélange (8) et, dans une seconde phase, du récipient de mélange(8) dans le récipient d'échantillonnage (4).

2. Dispositif suivant la revendication 1, caractérisé en ce qu'une vanne (5a, 5b) est disposée dans la conduite de prélèvement (3) avant et après le piquage de la conduite (6), respectivement.

3. Dispositif suivant la revendication 1, caractérisé en ce que la robinetterie (5a, 5b) constitue une vanne à plusieurs voies, et en ce que la conduite (6) est reliée à la conduite de prélèvement (3) par l'intermédiaire de la vanne à plusieurs voies (7).

4. Dispositif suivant l'une des revendications 1 à 3, caractérisé en ce que la conduite de transport (2) est une conduite de transport par dépression.

5. Dispositif suivant la revendication 4, caractérisé en ce qu'une conduite de retour (10) partant du récipient de mélange (8) est reliée à la conduite de transport par dépression (2) par l'intermédiaire d'une vanne (7).

6. Dispositif suivant la revendication 5, caractérisé en ce que la vanne (7) est une vanne à plusieurs voies, et en ce que la conduite (6), le récipient de mélange (8) et la conduite de retour (10) sont reliées entre eux par l'intermédiaire de la vanne (7).

7. Dispositif suivant l'une des revendications 1 à 6, caractérisé en ce que la pompe de dosage (9) est une pompe péristaltique.
